# EUROPEAN PATENT APPLICATION

(11) **EP 3 401 324 A1**
(43) Date of publication of application: **14.11.2018**
(21) Application number: 17868564.0
(22) Date of filing: 26.10.2017
(51) Int. Cl.: C07K 14/00, A61K 8/02, A61K 8/60, A61K 8/64, A61K 47/64, A61K 48/00, A61Q 19/00

(54) **NOVEL PROTEIN TRANSDUCTION DOMAIN AND USE THEREOF**

(30) Priority: 09.11.2016 KR 20160148578
(71) Applicant: Bioceltran Co., Ltd., Chuncheon-si, Gangwon-do 24232 (KR)
(72) Inventor: JANG, Sang Ho, Yongin-si Gyeonggi-do 16930 (KR); LEE, Gil Whoan, Chuncheon-si Gangwon-do 24209 (KR); HWANG, Jung Soon, Chuncheon-si Gangwon-do 24389 (KR); PARK, Jun Ho, Chuncheon-si Gangwon-do 24302 (KR); PARK, Ju Hee, Chuncheon-si Gangwon-do 24305 (KR)
(74) Representative: Rupp, Christian
(86) International application number: PCT/KR2017/011916
(87) International publication number: WO 2018/088733

(57) **Abstract**

The present invention relates to a novel protein transduction domain (PTD), which is a peptide having an amino acid sequence represented by SEQ ID NO: 1, and a use thereof. When the peptide represented by SEQ ID NO: 1 of the present invention is used as a PTD, biological molecules (target materials) such as proteins, peptides, DNA and the like can be easily introduced into cells in a high yield.

## Description

### [Technical Field]

The present invention relates to a novel protein transduction domain and use thereof, and more particularly, to a novel protein transduction domain (PTD) which is a peptide having an amino acid sequence represented by SEQ ID NO: 1 and use thereof.

### [Background Art]

Protein transduction domains (PTD) are peptides having 5 to 30 amino acid sequences which are useful for delivering peptides, proteins, DNA and the like. Recently, PTDs draw a great deal of attention in the fields of cosmetics, medicine and the like, since they can readily be joined to biological molecules (target substances), and do not affect structures or functions of biological molecules and are thus stable.

Among PTDs, amino acid sequences at positions 48 to 60 of HIV-derived TAT proteins were first known to have cell-permeable sequences and could deliver a variety of biological substances into cells. Then, after the mechanism of intracellular transduction was found, a number of sequences based on negatively charged cations which can be readily attached to cell membranes started to be produced.

PTDs known to date are broadly classified into the following three types: (i) amphipathic peptides (transportan, PEP1, MPG, pVEC, MAP and CADY); (ii) cationic peptides (polyarginines, TATs, penetratins and P22N); and (iii) hydrophobic peptides (K-FGF, C105Y). Amphipathic and cationic peptides are relatively well-known, but the number of hydrophobic peptides known to date is considerably limited because they are not obtained by artificial synthesis.

PTDs are capable of penetrating cells through which biological molecules cannot pass. In this regard, PTDs can incorporated into the cells through glycosaminoglycans (GAGs) of cell membranes or via an endocytosis pathway.

Bonding of PTDs to biological molecules (for example, siRNA, nucleic acids, small molecules, proteins, cytotoxic drugs, imaging agents and the like) can be carried out by a variety of methods including covalent bonding, ionic bonding, electrostatic attraction and the like.

The major advantages of PTDs, as compared to conventional delivery systems (for example, liposomes, polymers and the like), are low toxicity and less immune rejection response to treatment, when delivering biological molecules into cells for treatment or diagnosis. Based on this, it is possible to solve problems associated with high-concentration treatment with a stepwise treatment method.

Nevertheless, there are almost no PTDs used clinically and virus-derived PTDs disadvantageously affect immune response. For these reasons, there is an urgent need for carriers in the art that are not derived from viruses and have a high delivery efficiency.

### [Disclosure]

### [Technical Problem]

Therefore, the present invention has been made in view of the above problems, and it is an object of the present invention to develop and provide a novel protein transduction domain (PTD) which is capable of efficiently delivering a target material, while significantly reducing a variety of side effects.

### [Technical Solution]

In accordance with the present invention, the above and other objects can be accomplished by the provision of a peptide having an amino acid sequence represented by SEQ ID NO: 1.

In this case, the amino acid sequence represented by SEQ ID NO: 1 is preferably encoded by a nucleic acid sequence represented by SEQ ID NO. 2. In addition, the peptide is, for example, a protein transduction domain (PTD). In addition, the peptide is, for example, a gene delivery system.

Meanwhile, the present invention provides a conjugate formed by bonding a substance that can be chemically bonded to a peptide having an amino acid sequence represented by SEQ ID NO: 1, to the amino acid sequence represented by SEQ ID NO: 1. In this case, the chemical bond is, preferably, a covalent bond or a noncovalent bond. The substance that can be involved in the covalent bond is, for example, a peptide or protein. In addition, the noncovalent bond may be, for example, an ionic bond or an electrostatic attraction-based bond or a hydrophobic interaction-based bond. In addition, the substance that can be bonded via ionic bond or electrostatic attraction is, for example, a charged substance and the charged substance is, for example, DNA or RNA.

Meanwhile, the present invention provides a complex formed by mixing a substance that can be bonded via hydrophobic interaction or electrostatic attraction to a peptide having an amino acid sequence represented by SEQ ID NO: 1, with the amino acid sequence represented by SEQ ID NO: 1. In this case, the substance that can be chemically bonded via electrostatic attraction is, for example, a charged substance and the charged substance is, for example, DNA or RNA.

Meanwhile, the present invention provides a cosmetic composition including a conjugate formed by bonding a substance that can be chemically bonded to a peptide having an amino acid sequence represented by SEQ ID NO: 1, to the amino acid sequence represented by SEQ ID NO: 1, or a complex formed by mixing a substance that can be bonded via hydrophobic interaction or electrostatic attraction to a peptide having an amino acid sequence represented by SEQ ID NO: 1, with the amino acid sequence represented by SEQ ID NO: 1.

Meanwhile, the present invention provides a pharmaceutical composition including a conjugate formed by bonding a substance that can be chemically bonded to a peptide having an amino acid sequence represented by SEQ ID NO: 1, to the amino acid sequence represented by SEQ ID NO: 1, or a complex formed by mixing a substance that can be bonded via hydrophobic interaction or electrostatic attraction to a peptide having an amino acid sequence represented by SEQ ID NO: 1, with the amino acid sequence represented by SEQ ID NO: 1.

Meanwhile, the present invention provides a method for introducing, into cells in a mammal, a substance that can be chemically bonded to a peptide having an amino acid sequence represented by SEQ ID NO: 1, including bonding the peptide having an amino acid sequence represented by SEQ ID NO: 1 to the substance that can be chemically bonded to the peptide having an amino acid sequence represented by SEQ ID NO: 1, to introduce, into the cells, the substance that can be chemically bonded to the peptide having an amino acid sequence represented by SEQ ID NO: 1.

Meanwhile, the present invention provides a method for introducing, into cells in a mammal, a substance that can be bonded via hydrophobic interaction or electrostatic attraction to a peptide having an amino acid sequence represented by SEQ ID NO: 1, including bonding the peptide having an amino acid sequence represented by SEQ ID NO: 1 to the substance that can be bonded via hydrophobic interaction or electrostatic attraction to the peptide having an amino acid sequence represented by SEQ ID NO: 1, to introduce, into the cells, the substance that can be bonded via hydrophobic interaction or electrostatic attraction to the peptide having an amino acid sequence represented by SEQ ID NO: 1.

### [Advantageous Effects]

When the peptide represented by SEQ ID NO: 1 of the present invention is used as a PTD, advantageously, biological molecules (target materials) such as proteins, peptides, DNA and the like can be easily introduced into cells in a high yield.

### [BRIEF DESCRIPTION OF THE DRAWINGS]

The above and other objects, features and other advantages of the present invention will be more clearly understood from the following detailed description taken in conjunction with the accompanying drawings, in which:
FIG. 1 is a map of a general GFP expression vector;
FIG. 2 is a map of a TAT-GFP expression vector;
FIG. 3 is a map of a PEP1-GFP expression vector;

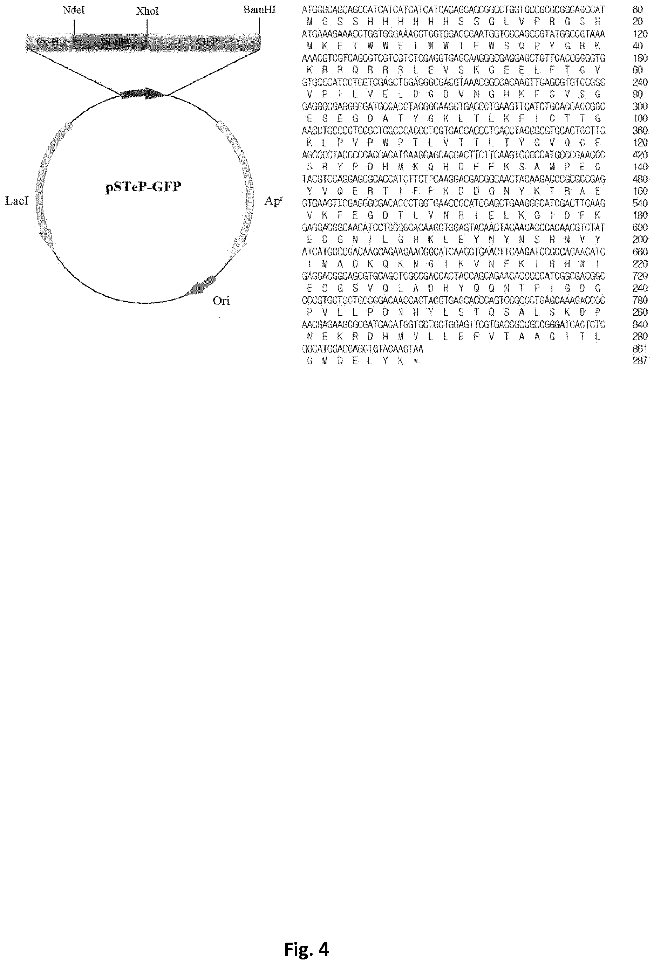

FIG. 4 is a map of a STeP-GFP expression vector;
FIG. 5 shows expression results of GFP recombinant proteins;
FIG. 6 shows purification results of GFP recombinant proteins;
FIG. 7 shows comparison results of cell penetration capability of GFP proteins;
FIG. 8 shows comparison results of skin penetration capability of GFP proteins;
FIG. 9 is a map of a PEP1-EGF expression vector;
FIG. 10 is a map of a STeP-EGF expression vector;
FIG. 11 shows expression results of EGF recombinant proteins (PEP1-EGF, STeP-EGF);
FIG. 12 shows purification results of the EGF recombinant proteins (PEP1-EGF, STeP-EGF);
FIG. 13 shows cell proliferation efficacy of EGF recombinant proteins (EGF, PEP1-EGF, STeP-EGF);
FIG. 14 shows comparison results of skin penetration capability of EGF proteins;
FIG. 15 shows evaluation results of cytotoxicity of cell-penetrating peptides;
FIG. 16 shows comparison results of cell penetration capability of a cell-penetrating peptide-FITC mixture;
FIG. 17 shows comparison results of skin penetration capability of the cell-penetrating peptide-FITC mixture;
FIG. 18 shows comparison results of cell penetration capability of a cell-penetrating peptide/FITC-DDBD mixture; and
FIG. 19 shows comparison results of skin penetration capability of a cell-penetrating peptide-FGF2 mixture.

### [Best Mode]

A number of research and patent documents are referenced throughout the present specification and citations thereof are disclosed. The cited research and patent documents are incorporated by reference in their entirety herein so that the levels of the technical field to which the present invention pertains and contents of the present invention can be more clearly described.

As a result of developing peptides that are capable of more efficiently delivering target materials into cells and drug delivery systems using the same, the present inventors developed the cell-penetrating peptide represented by SEQ ID NO: 1.

The present invention was completed based on the finding that the peptide represented by SEQ ID NO: 1 is amphipathic and can be efficiently applied to deliver target substances into cells.

Based on these results, the present invention provides a peptide having an amino acid sequence represented by SEQ ID NO: 1. In this case, the amino acid sequence represented by SEQ ID NO: 1 is preferably encoded by a nucleic acid sequence represented by SEQ ID NO. 2. In addition, a peptide to realize the desired purpose may be linked to the N-terminal or C-terminal of the amino acid sequence, depending on the desired purpose.

The peptide represented by SEQ ID NO: 1 of the present invention may be used as a protein transduction domain (PTD) or a gene delivery system.

Meanwhile, the present invention provides a conjugate formed via chemical bonding between a substance that can be chemically bonded to a peptide having an amino acid sequence represented by SEQ ID NO: 1, and the amino acid sequence represented by SEQ ID NO: 1.

The term "chemical bond" as used herein includes all kinds of chemical bonds, and is for example a covalent bond or a noncovalent bond. The covalent bond, for example, means that a target substance (i.e., substance to be introduced into cells or the skin) can be linked via a peptide bond to the N-terminal or C-terminal of the amino acid sequence represented by SEQ ID NO: 1 according to the present invention. In this case, the target substance is for example a peptide or protein that exerts a certain function such as skin cosmetic efficacy or medical efficacy. Examples of the peptide include dipeptide diaminobutyroyl benzylamide diacetate (SYN-AKE®), acetyl hexapeptide-8 (Argireline®) and the like. Examples of the protein include epidermis growth factors (EGFs), fibroblast growth factor-1 (FGF1), fibroblast growth factor-2 (FGF2), superoxide dismutase-1 (SOD1), catalase (CAT), FK506-binding proteins (FK506bps), botulinum neurotoxin type A light chain (BonT/A Light chain) and the like.

Meanwhile, regarding the conjugate of the present invention, the noncovalent bond is, for example, an ionic bond or an electrostatic attraction-based bond or a hydrophobic interaction-based bond. A variety of biological molecules (for example, siRNA, nucleic acids, small molecules, proteins, cytotoxic drugs, imaging agents and the like) can be bonded via the ionic bond or electrostatic attraction or hydrophobic interaction to the peptide represented by SEQ ID NO: 1 of the present invention and can be introduced into cells or the skin. The substance that can participate in the ionic bond or electrostatic attraction-based bond is, for example, a charged substance and the charged substance is, for example, DNA or RNA.

Meanwhile, the present invention provides a complex formed by mixing a substance that can be bonded via hydrophobic interaction or electrostatic attraction to a peptide having an amino acid sequence represented by SEQ ID NO: 1, with the amino acid sequence represented by SEQ ID NO: 1. It was confirmed that, according to the present invention, only by mixing, rather than inducing an artificial chemical bond, the target substance that can be bonded to the peptide represented by SEQ ID NO: 1 via hydrophobic interaction or electrostatic attraction according to the present invention can be bonded to the peptide represented by SEQ ID NO: 1 of the present invention via hydrophobic interaction or electrostatic attraction and thus can be introduced into cells or the skin. In this case, the substance that can be bonded based on the electrostatic attraction is, for example, a charged substance and the charged substance is, for example, DNA or RNA.

Meanwhile, the present invention provides a cosmetic composition including a conjugate formed by bonding a substance that can be chemically bonded to a peptide having an amino acid sequence represented by SEQ ID NO: 1, to the amino acid sequence represented by SEQ ID NO: 1, or a complex formed by mixing a substance that can be bonded via hydrophobic interaction or electrostatic attraction to a peptide having an amino acid sequence represented by SEQ ID NO: 1, with the amino acid sequence represented by SEQ ID NO: 1. The following tests according to the present invention proved that the conjugate or complex produced according to the present invention is capable of effectively penetrating the skin.

The cosmetic composition of the present invention is not limited to a certain form (formulation) and includes all formulations. For example, the cosmetic composition is preferably any one selected from: skin care formulations consisting of skin toner, gels, water-soluble liquids, creams, essences, oil-in-water (O/W) and water-in-oil (W/O) emulsions and ointments; and color makeup formulations consisting of oil-in-water (O/W) and water-in-oil (W/O) makeup bases, foundations, skin covers, lipsticks, lip glosses, face powders, two-way cakes, eye shadows, cheek colors and eyebrow pencils.

In addition, the cosmetic composition of the present invention may include one or more additives ordinarily used in the field of cosmetics, for example, hydrophilic or lipophilic gelling agents, hydrophilic or lipophilic activators, preservatives, antioxidants, solvents, fragrances, fillers, blockers, pigments, odor-absorbers and dyes.

Meanwhile, the present invention provides a pharmaceutical composition including a conjugate formed by bonding a substance that can be chemically bonded to a peptide having an amino acid sequence represented by SEQ ID NO: 1, to the amino acid sequence represented by SEQ ID NO: 1, or a complex formed by mixing a substance that can be bonded via hydrophobic interaction or electrostatic attraction to a peptide having an amino acid sequence represented by SEQ ID NO: 1, with the amino acid sequence represented by SEQ ID NO: 1.

The pharmaceutical composition of the present invention is not limited to a certain form (formulation) and includes all formulations. Specific examples of the formulation include plasters, granules, lotions, liniments, lemonades, aromatic waters, powders, syrups, ophthalmic ointments, liquids and solutions, aerosols, extracts, elixirs, ointments, fluid extracts, emulsions, suspensions, decoctions, infusions, ophthalmic solutions, tablets, suppositories, injections, spirits, cataplasm, capsules, creams, troches, tinctures, pastes, pills, and soft and hard gelatin capsules.

In addition, the pharmaceutical composition of the present invention may further include pharmaceutically acceptable carriers, diluents or excipients. Examples of useful carriers, diluents or excipients include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearate and mineral oils. These ingredients may be used alone or in combination of two or more thereof.

Meanwhile, the present invention provides a method for introducing, into cells in a mammal, a substance that can be chemically bonded to a peptide having an amino acid sequence represented by SEQ ID NO: 1, including bonding the peptide having an amino acid sequence represented by SEQ ID NO: 1 to the substance that can be chemically bonded to the peptide having an amino acid sequence represented by SEQ ID NO: 1 to introduce, into the cells, the substance that can be chemically bonded to the peptide having an amino acid sequence represented by SEQ ID NO: 1.

In addition, the present invention provides a method for introducing, into cells in a mammal, a substance that can be bonded via hydrophobic interaction or electrostatic attraction to a peptide having an amino acid sequence represented by SEQ ID NO: 1, including bonding the peptide having an amino acid sequence represented by SEQ ID NO: 1 to the substance that can be bonded via hydrophobic interaction or electrostatic attraction to the peptide having an amino acid sequence represented by SEQ ID NO: 1 to introduce, into the cells, the substance that can be bonded via hydrophobic interaction or electrostatic attraction to the peptide having an amino acid sequence represented by SEQ ID NO: 1.

Through the method of the present invention, a target substance to be introduced into cells can be easily introduced into cells at a high yield. In this case, apart from using the peptide having an amino acid sequence represented by SEQ ID NO: 1 according to the present invention, the technology required to introduce the target substance into cells may be any method well-known in the art.

As described above, the present invention suggests that the peptide having 27 amino acid sequences represented by SEQ ID NO: 1 is capable of penetrating cells, and delivering proteins and genes. In addition, the present invention is considered to be basic data that suggests a method for effectively delivering substances using PTDs since the delivery efficiency depends on the position of the delivered substance. In addition, the peptide represented by SEQ ID NO: 1 according to the present invention can be useful as a delivery system owing to the benefit of the capability to deliver substances only by simple mixing.

The specific features of the present invention have been described in detail and it would be obviously understood by those having ordinary knowledge in the art that such specific description is provided only as an embodiment and should not be construed as limiting the scope of the present invention. Accordingly, the substantial scope of the present invention should be defined only by the attached claims and equivalents thereto.

### [Example 1. Production of cell- and skin-permeable GFP protein expression vectors]

In order to easily deliver macromolecular proteins or peptides into cells and the skin, recombinant protein expression vectors were produced by fusing peptides that are capable of penetrating the cells and skin with proteins (cargo) to be delivered.

In the present Example, in order to easily analyze the capability of such fusion proteins to deliver a target material into cells and the skin, a green fluorescence protein (hereinafter, simply referred to as "GFP") was selected as the target protein (cargo) to be delivered.

Fusion proteins obtained by fusing a conventionally well-known PTD, *i*.*e*., TAT or PEP1 with GFP were compared with the STeP (SEQ ID NO: 1) newly developed by the present invention in terms of the capability to deliver a target material into cells and the skin.

### (1) Production of pGFP vector

DNA fragments corresponding to base sequences of GFP were subcloned at positions XhoI and BamHI of plasmid pET15b to produce pGFP expressing GFP proteins. Since cell- and skin-penetrating peptides were not fused with the pGFP expression vector and the GFP was a hydrophilic polymer protein of about 29 kDa, GFP expressed in this vector was considered to have no capability to deliver a target material into cells and the skin. For this reason, this was produced as a control group to compare the capability to penetrate cells and skin.

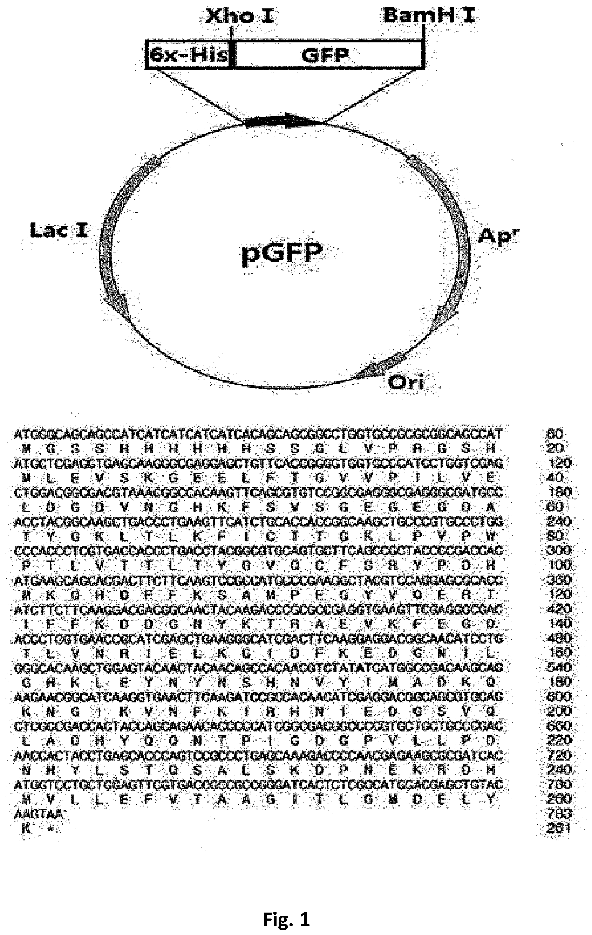

The complete sequences of GFP were amplified from DNA fragments corresponding to the base sequences of GFP by polymerase chain reaction (PCR) with a Pfu DNA polymerase using the plasmid pEGFP-C2 (Clontech). The sequence of a sense primer is 5'-CTCGAGGTGAGCAAGGGCGAGGAGCTG-3' and the sequence of an antisense primer is 5'-GGATCCTTACTTGTACAGCTCGTCC ATGCCGAG-3'. The PCR product was cut with XhoI-Bam HI and subcloned at the position of XhoI-Bam HI of pET15b (Invitrogen, Carlsbad, CA) to produce pGFP expressing general GFP proteins. FIG. 1 is a map of a general GFP expression vector.

### (2) Production of pTAT-GFP vector

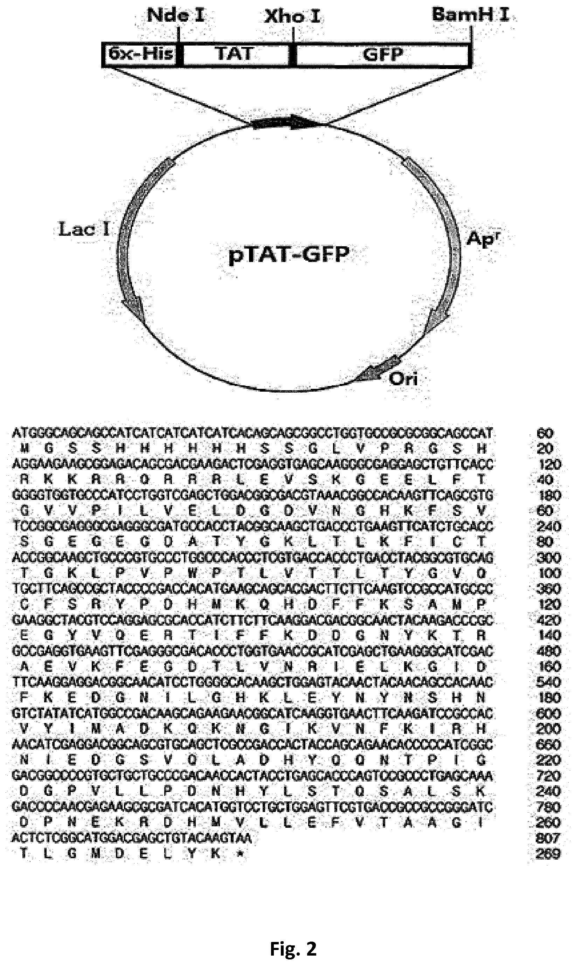

pTAT-GFP expressing HIV-1 TAT-fused GFP was produced by the following method. First, two oligonucleotides were produced and were synthesized into double stranded oligonucleotides encoding 9 amino acids of HIV-1 TAT. The sequence encoding 9 amino acids of HIV-1 TAT includes a top strand, 5'-TAGGAAGAAGCGGAGACAGCGACGAAGAC-3' and a bottom strand, 5'-TCGAGTCTTCGTCGCTGTCTCCGCTTCTTCC-3'. Double stranded oligonucleotides were directly subcloned into NdeI-XhoI of pGFP to produce pTAT-GFP expressing TAT-GFP proteins. FIG. 2 is a map of the TAT-GFP expression vector.

### (3) Production of pPEP1-GFP vector

pTAT-GFP expressing PEP1-fused GFP was produced by the following method. First, two oligonucleotidees, that is, the top strand 5'-TATGAAAGAAACCTGGTGGGAAACCTGGTGGACCGAATGGAGCCAGCCGAAAAAAAAACG TAAAGTGC-3' and the bottom strand 5'-TCGAGCACTTTACGTTTTTTTTTCGGCTGAGACCATTCGGTCCACCAGGTTTCCCACCAG GTTTCTTTCC-3' were synthesized to produce double stranded oligonucleotides encoding PEP1 peptides.

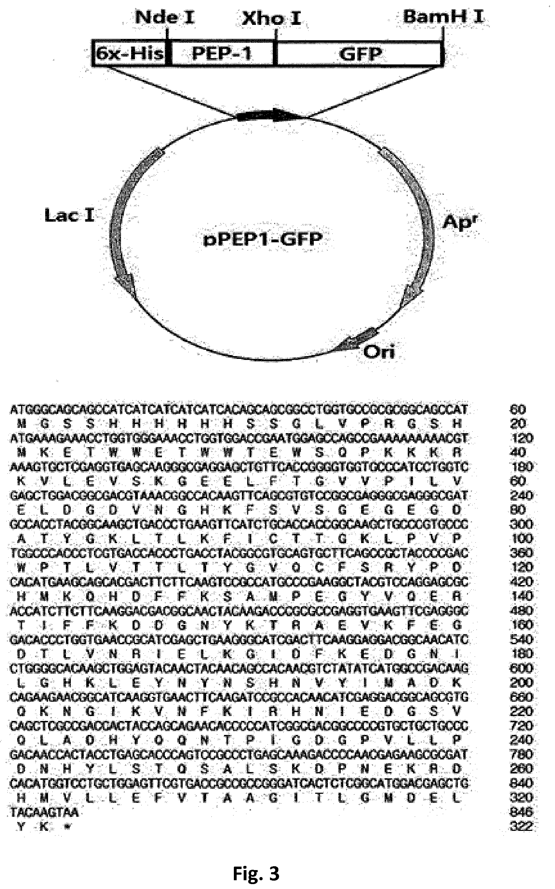

The double stranded oligonucleotides were directly subcloned into NdeI-XhoI of pGFP to produce pPEP1-GFP expressing PEP1-GFP proteins. FIG. 3 is a map of a PEP1-GFP expression vector.

### (4) Production of pSTeP-GFP vector

pSTeP-GFP expressing STeP-fused GFP was produced by the following method. First, two oligonucleotides, that is, top strand 5'-TATG AAAGAAACCTGGTGGGAAACCTGGTGGACCGAATGGTCCCAGCCGTATGGCCGTAAAAAA CGTCGTCAGCGTCGTCGTC-3' and bottom strand 5'-TCGAGACGACGACGCTGACGACGTTTTTTACGGCCATACGGCTGGGACCATTCGGTCCAC CAGGTTTCCCACCAGGTTTCTTTCC-3' were synthesized to produce double stranded oligonucleotides encoding STeP peptides. The double stranded oligonucleotides were directly subcloned into NdeI-XhoI of pGFP to produce STeP-GFP expressing STeP-GFP proteins. FIG. 3 is a map of a STeP-GFP expression vector.

### [Example 2. Expression of GFP recombinant proteins]

In the present Example, the corresponding proteins were expressed from the recombinant vectors produced in Example above. Four types of expression vectors were each transformed into *E*. *coli* Rosetta-gami 2(DE3) cells. The transformed bacterial cells were cultured at 37°C in 1,000 mL of LB medium containing ampicillin until OD₆₀₀ reached 0.6 and were further cultured using 0.5 mM IPTG at 22°C for 24 hours to induce expression of GFP proteins.

Whether or not respective GFP proteins were expressed was identified by collecting cultured cells before and after inducing expression of target proteins by addition of IPTG, conducting SDS-PAGE electrophoresis and then dyeing the proteins with Coomassie brilliant blue.

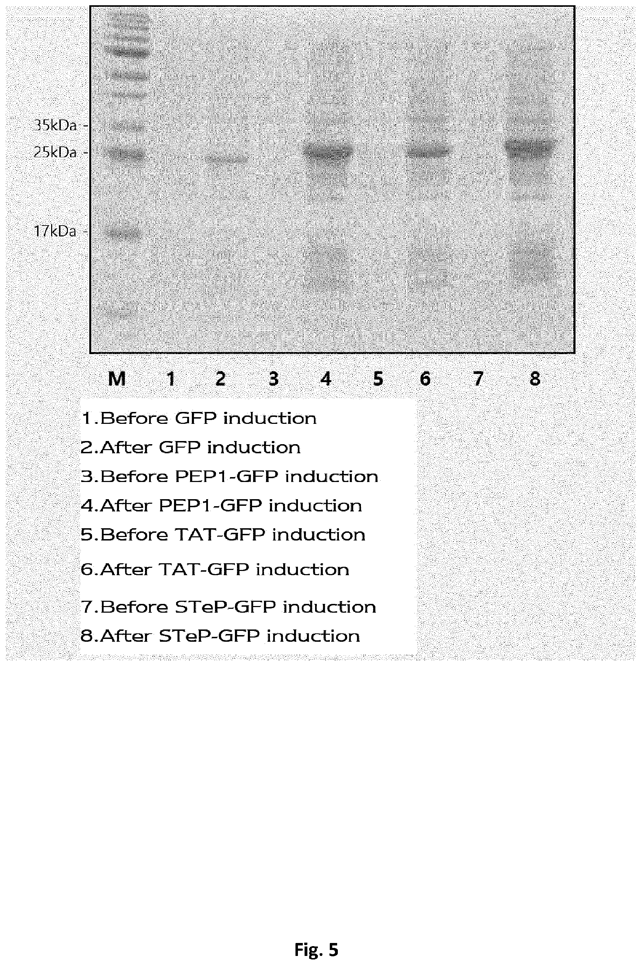

As a result, as can be seen from FIG. 5, GFP expresses 29.35 kDa proteins, TAT-GFP expresses 30.54 kDa proteins, PEP1-GFP expresses 32.18 kDa proteins, and STeP-GFP expresses 32.95 kDa proteins. FIG. 5 shows expression results of GFP recombinant proteins.

### [Example 3. Purification of GFP recombinant proteins]

In the present Example, the proteins each expressed in Example were subjected to cell disruption and then only GFP proteins were purely isolated and purified using His tag-affinity columns. Four types of GFP proteins were purified in the same manner as below.

pET15-GFP, pTAT-GFP, pPEP1-GFP and pSTeP-GFP transformed cells were lysed with a sonicator in 1X native buffer (50 mM NaPO₄, 0.5 M NaCl), then centrifuged and separated into an agglomerate (inclusion body) and the cytoplasm (supernatant).

All four types of GFP proteins were His₆-tagged and, using this feature, each supernatant separated by centrifugation after cell disruption was purified with a Ni²⁺-NTA affinity column (Ni²⁺-nitrilotriacetic acid Sepharose affinity column, GE Healthcare, USA).

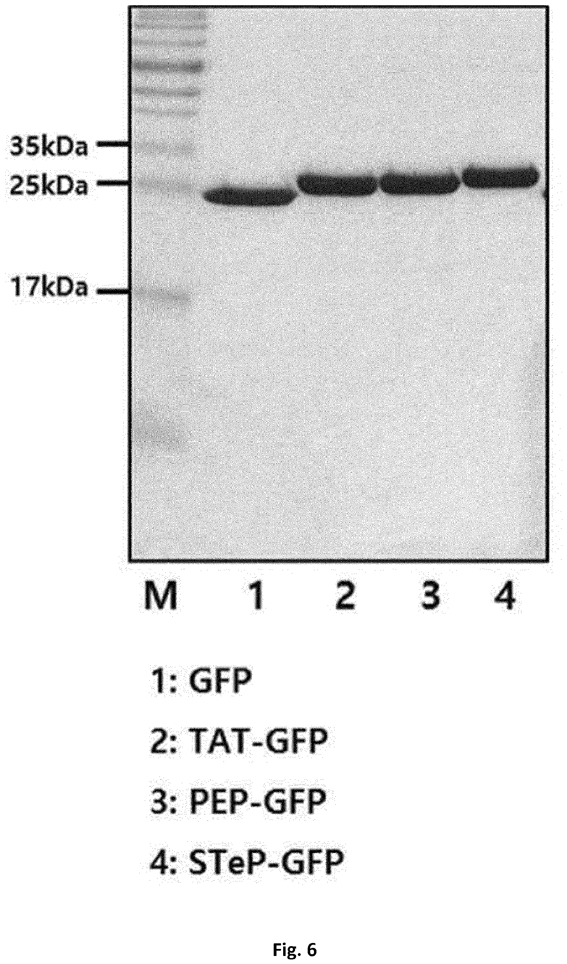

Equilibrium of columns and bonding with GFP proteins were carried out using 1X native buffer, and washing and elution were carried out using solutions of different concentrations of imidazole in 1X native buffer. Purely isolated and purified GFP proteins were subjected to dialysis using stabilized buffer (50 mm NaPO₄, 0.1M NaCl, 10% Glycerol, pH 7.5). The final products thus obtained are referred to as "GFP", "Tat-GFP", "PEP1-GFP" and "STeP-GFP". FIG. 6 shows purification results of GFP recombinant proteins.

### [Example 4. Analysis of cell penetration of cell- and skin-penetrating GFP proteins]

In the present Example, in an attempt to compare cell penetration capability of four types of GFP proteins purified in Example, cell penetration was observed using GFP green fluorescence in HaCaT (human keratinocyte) cells.

HaCaT (human keratinocyte) cells were cultured on a slide for culture for 24 hours. After the culture medium was removed, the cells were washed with HBSS three times and then treated with GFP and cell-penetrating GFP proteins (PEP1-GFP, TAT-GFP, STeP-GFP). Upon treatment of cells, stocks were prepared such that respective proteins reached 10 mM and diluted at 1/10 in a medium, and cells were treated such that the final concentration reached 10 µM and then cultured for 2 hours. After 2 hours, the medium was removed, and cells were washed with PBS and then immobilized at -20°C using cooling methanol for 10 minutes. The cells were thoroughly washed with PBS and dyed with a solution of a nuclear staining reagent (DAPI) diluted to 1/5000 in PBS in the absence of light for 10 minutes. The resulting cells were thoroughly washed with PBS, mounted and then analyzed using a confocal scanning laser microscope.

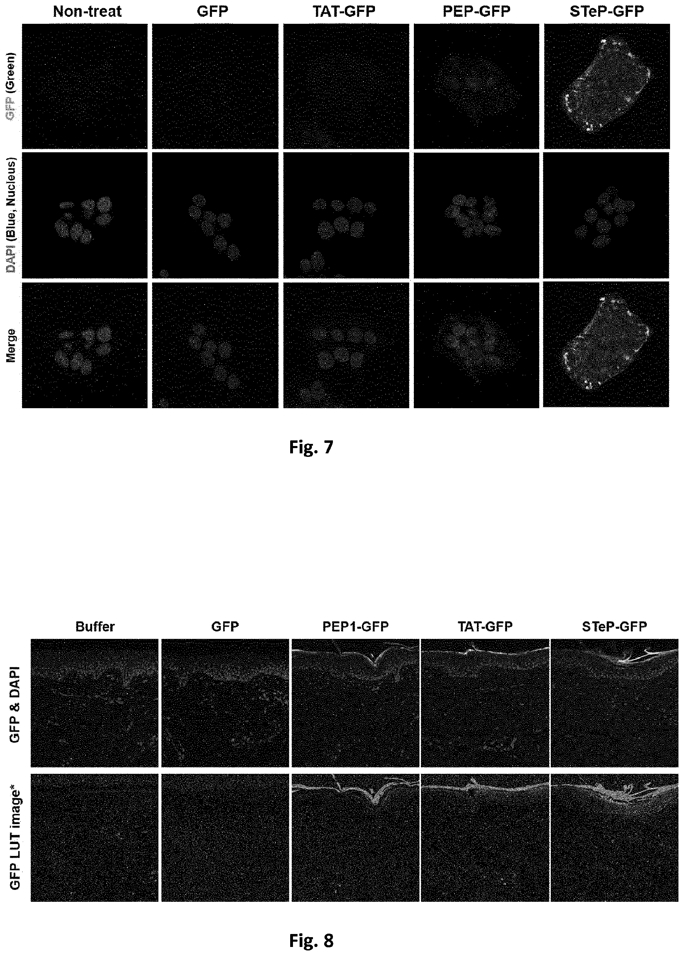

Results showed that the group treated with STeP-GFP proteins exhibited the brightest green fluorescence in cells, indicating that STeP greatly improved the effect of penetration into cells. FIG. 7 shows comparison results of cell penetration capability of GFP proteins.

### [Example 5. Analysis of skin penetration of cell- and skin-penetrating GFP proteins]

In order to identify whether or not STeP can improve penetration of GFP proteins into the skin, as well as into the cells, Franz cells (diameter of 9 mm) were used.

The porcine skin (0.7 mm thickness, Medikinetics) was mounted between the top and bottom of the cells, and the top was treated with 300 µl of 50 µM GFP proteins. After 24 hours, the porcine skin was isolated and washed with PBS three times. After washing, the cryo mold was treated with an OCT embedding matrix (Cell Path Ltd., UK) and tissues were frozen at -70°C. The frozen tissues were cross-sectioned to a thickness of 7 µm at -20°C using a cryotome (HM520, Germany) to produce fragment slides. To dye the nucleus of the skin cells, tissue fragments fixed at a slide glass were dyed with a solution of a nuclear staining reagent (DAPI) diluted at 1/5000 in PBS in the absence of light for 10 minutes and washed with PBS three times. The resulting cells were mounted and then analyzed with a confocal scanning laser microscope.

Test results showed that GFP was not delivered into porcine skin tissues at all, while the STeP-GFP of the present invention could be efficiently delivered into the skin. FIG. 8 shows comparison results of skin penetration capability of GFP proteins. In FIG. 8, 'LUT Image' is an image with an improved contrast using look up table (LUT).

### [Example 6. Production of cell- and skin-penetrating EGF protein expression vectors]

GFP proteins were used in order to easily observe cell and skin-penetration capability of STeP, but recombinant protein expression vectors were produced by fusing epidermal growth factors (EGF) with STeP as another example to describe that the effect of STeP is not limited to GFP.

Epidermal growth factors are hormones facilitating cell division of epidermal cells, are peptides having 53 amino acid residues and have a molecular weight of 6,200 Dalton. The epidermal growth factors are polypeptide proteins that have three disulfide bonds (Cys6-Cys20, Cys14-Cys31 and Cys33-Cys42) that play a pivotal role in physiological actions.

Epidermal cell growth factors are known as factors that act to proliferate epidermal cells *in situ* and cure wounds. Epidermal growth factors are known to be used as medicines for treating wounds in the skin or cornea, or medicines for treating stomach ulcers owing to activities of facilitating mitosis, of facilitating cell growth and of inhibiting secretion of gastric acid in a variety of cells including epidermal cells and mesenchymal cells.

The substances obtained by fusing each of PEP1 and STeP with EGF were compared with newly developed STeP in terms of cell proliferation efficacy and delivery into the skin.

### (1) Production of pPEP1-EGF vectors

In order to produce skin-penetrating human epidermal growth factors (rhEGFs), human epidermal growth factor-fused protein expression vectors were produced.

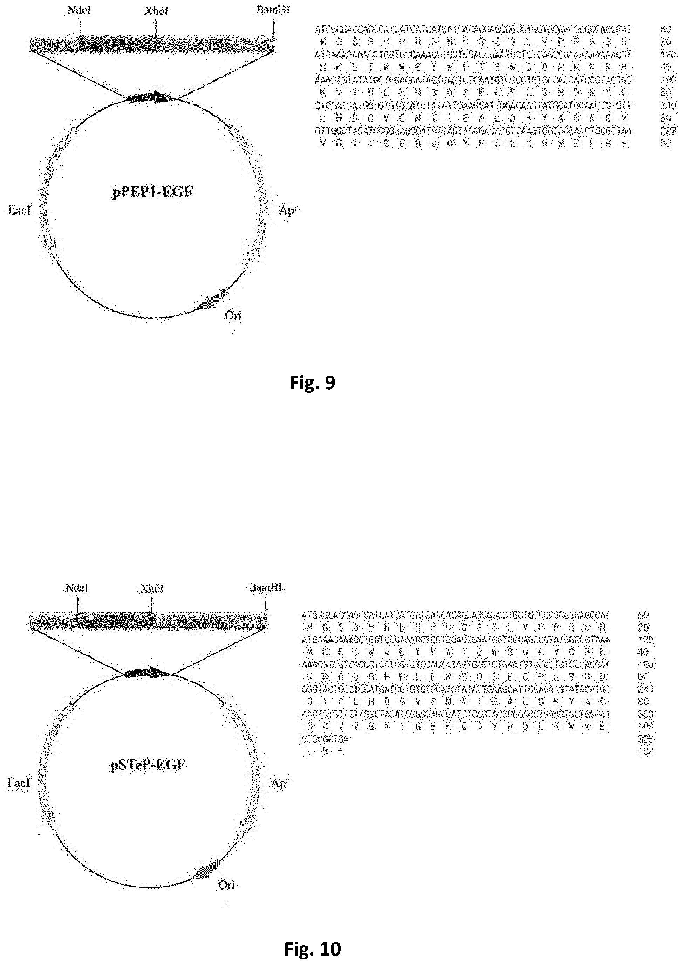

Protein expression vectors for expressing fusion proteins of human epidermal growth factors and PEP1 (KETWWETWWTEWSQPKKKRKV) peptides were produced. Two primers of the human epidermal growth factors were synthesized based on cDNA sequences. The sense primer 5'-ACACTCGAGAATAGTGACTCTGAATGTC-3' included XhoI restriction enzyme cleavage sites and the antisense primer 5'-TGTGGATCCTTAGCGCAGTTCC-3' included BamHI restriction enzyme cleavage sites. Polymerase chain reaction (PCR) was conducted and the polymerase chain reaction product was linked to a TA-cloning vector. Then, the PCR product was cut with XhoI and BamHI, and the cut oligonucleotides were eluted (Invitek, Berlin, German). The cut oligonucleotides were linked to the pET15b vector using a T4 DNA ligase (Takara, Otsu, Shiga, Japan) and transformed into *E.coli* DH5 cells. The pEGF vector was purified from the transformed bacteria. The top strand 5'-GGGGAATTCCATATGAAAGAAACCTGGTGGGAAACCTGGTGGACCGAATGGTCTCAGCCG AAAAAAAAACGTAAAGTGCCTCGAGTATAT-3' and bottom strand 5'-ATATACTCGAGGCACTTTACGTTTTTTTTTCGGCTGAGACCATTCGGTCCACCAGGTTTC CCACCAGGTTTCTTTCATATGGAATTCCCC-3' were synthesized and the resulting product was annealed to produce double stranded oligonucleotides encoding Pep-1 peptides. The double stranded oligonucleotides were directly subcloned into NdeI-XhoI of pEGF to produce pPEP1-EGF expressing PEP1-EGF proteins. FIG. 9 is a map of a PEP1-EGF expression vector.

### (2) Production of pSTeP-EGF vector

pSTeP-GFP expressing STeP-fused GFP was produced by the following method. First, two oligonucleotides, that is, top strand 5'-TATG AAAGAAACCTGGTGGGAAACCTGGTGGACCGAATGGTCCCAGCCGTATGGCCGTAAAAAA CGTCGTCAGCGTCGTCGTC-3' and bottom strand 5'-TCGAGACGACGACGCTGACGACGTTTTTTACGGCCATACGGCTGGGACCATTCGGTCCAC CAGGTTTCCCACCAGGTTTCTTTCC-3' were synthesized to produce double stranded oligonucleotides encoding STeP peptides. The double stranded oligonucleotides were directly subcloned into NdeI-XhoI of pGFP to produce STeP-GFP expressing STeP-GFP proteins. FIG. 10 is a map of a STeP-GFP expression vector.

### [Example 7. Expression of EGF recombinant proteins (PEP1-EGF, STeP-EGF)]

In the present Example, the corresponding proteins were expressed from the recombinant vectors produced in Example above. A commercially available general EGF was used to compare with cell- and skin-penetrating EGFs (PEP1-EGF, STeP-EGF) in terms of efficacy and effects, and in the present Example, expression and purification from vectors were omitted. The commercially available general EGF was a protein of about 6 kDa which had no function of delivery into cells and the skin.

Two types of expression vectors were each transformed into *E. coli* BL21 (DE3) cells. The transformed bacterial cells were cultured at 37°C in 1,000 mL of LB medium containing ampicillin until OD₆₀₀ reached 0.6 and were further cultured using 0.5 mM IPTG at 37°C for 8 hours to induce expression of PEP1-EGF and STeP-EGF proteins.

Whether or not respective EGF proteins were expressed was identified by collecting cultured cells before and after inducing expression of target proteins depending on addition of IPTG, conducting SDS-PAGE electrophoresis and then dyeing the proteins with Coomassie brilliant blue.

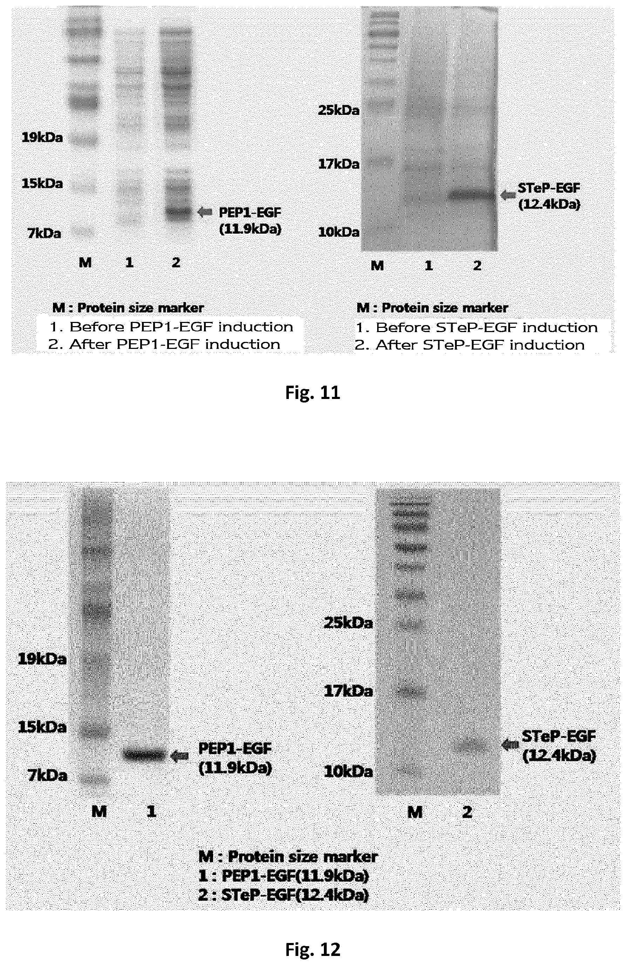

As a result, as can be seen from FIG. 11, PEP1-EGF expresses 11.9 kDa proteins and STeP-EGF expresses 12.4 kDa proteins. FIG. 11 shows expression results of EGF recombinant proteins (PEP1-EGF, STeP-EGF).

### [Example 8. Pure isolation and purification of EGF recombinant proteins (PEP1-EGF, STeP-EGF)]

In the present Example, the proteins each expressed in Example were subjected to cell disruption and then only PEP1-EGF and STeP-EGF proteins were purely isolated and purified using His tag-affinity columns. Two types of EGF proteins were purified in the same manner as below.

pPEP1-EGF and pSTeP-EGF transformed cells were cultured and expressed, and the harvested cells were lysed in 50 mM Tris buffer (50 mM Tris, 0.5 M NaCl, 2% Triton X-100, pH 8.0) with a sonicator, and then centrifuged and separated into an agglomerate (inclusion body) and the cytoplasm (supernatant).

Two types of EGF proteins were expressed in the form of an agglomerate and most of the target proteins was incorporated in the agglomerate after cell disruption and then centrifugation. Accordingly, the target protein should be solubilized using 8M urea buffer for pure isolation and purification.

In addition, two types of GFP proteins were His₆-tagged and, using this feature, the supernatant each separated by centrifugation after cell disruption was purified with a Ni²⁺-NTA affinity column (Ni²⁺-nitrilotriacetic acid Sepharose affinity column, GE Healthcare, USA).

Respective agglomerates collected by centrifugation after cell disruption were solubilized in 50 mM Tris buffer (50 mM Tris, 8 M urea, pH 8.0) containing 8M urea and were centrifuged to separate into insoluble impurities and the supernatant containing respective EGF proteins.

The supernatants containing EGF proteins were purified with a Ni²⁺-NTA affinity column (Ni²⁺-nitrilotriacetic acid Sepharose affinity column, GE Healthcare, USA). Equilibrium of columns and bonding with EGF proteins were carried out using 50 mM Tris buffer (50 mM Tris, 8M urea, pH 8.0), and washing and elution were carried out using solutions of different concentrations of imidazole in conjugation buffer. Purely isolated and purified proteins were referred to as "PEP1-EGF" and "STeP-EGF".

### [Example 9. Refolding and HPLC purification of EGF recombinant proteins (PEP1-EGF, STeP-EGF)]

Two types of purely isolated and purified proteins were further subjected to a refolding process to impart activity thereto. Refolding of the EGF proteins was carried out in the same manner as below.

The purely isolated and purified proteins were each diluted at 10X in refolding reduction buffer (50 mM Na₂CO₃, 1 mM L-Cysteine, pH 9.6) with a stirrer and were further reacted at 25°C for one hour. Then, for refolding oxidation, L-Cystine was added such that a final concentration reached 5 mM and reaction was conducted at 4°C for 15 hours with a stirrer. The precipitate produced during refolding was removed by centrifugation from the completely reacted refolding solution and the resulting refolding supernatant was concentrated and purified by HPLC.

Refolded and then concentrated EGF proteins were acidized with 6M HCl (pH 2.7 to 3.0) and filtered through a 0.2 µm filter to prepare samples, and the samples were primarily purified by MPLC.

The mobile phase used herein was water with 0.1% trifluoroacetic acid (TFA) (v/v) and acetonitrile (ACN), and the column used herein was a C18 40g cartridge. The samples were purified using a UV detector with a stepwise gradient of increasing acetonitrile (ACN) concentration and then analyzed by HPLC. As a result, EGF and STeP-EGF proteins having a purity of 50 to 80% were collected from peaks corresponding to PEP1-EGF and STeP-EGF proteins, and concentrated in a rotary evaporator.

In addition, the PEP-EGF and STeP-EGF proteins having a high purity of 95% or more were further secondarily purified by HPLC. HPLC was carried out using, as mobile phases, water with 0.1% trifluoroacetic acid (TFA) (v/v) and acetonitrile (ACN) and a C18 50×250 mm column. Proteins having a purity of 95% or more were obtained using a UV detector with a stepwise gradient of increasing acetonitrile (ACN) concentration, concentrated and dialyzed using stabilization buffer (50 mm Na₂CO₃, 10% Glycerol, pH 9.6). FIG. 12 shows purification results of EGF recombinant proteins (PEP1-EGF and STeP-EGF).

### [Example 10. Measurement of cell proliferation activity of EGF recombinant proteins (PEP1-EGF, STeP-EGF)]

In order to evaluate cell proliferation effects of EGF, PEP1-EGF and STeP-EGF, mouse fibroblasts (Balb/c 3T3 clone A31 cells) were treated with respective purified proteins at different concentrations and cell growth rate was measured. The mouse fibroblasts were cultured in a growth medium for 24 hours, subjected to starvation in serum-free medium for 20 hours, treated at different concentrations with respective recombinant proteins and then cultured for 24 hours. More specifically, Balb/c 3T3 clone A31 cells were seeded at a density of 10⁴ cells/well on a 96-well plate, cultured in 100 µl of DMEM (growth medium) containing 10% BCS for 24 hours, transferred to a 0% BCS medium (serum-free medium) and subjected to starvation for about 20 hours. Then, EGF, PEP1-EGF and STeP-EGF proteins were prepared by diluting 3X at the maximum concentration of 90 ng/ml and each treated on the wells for 24 hours.

The final measurement was carried out using a WST assay (Takara, Otsu, Shiga, Japan) model in accordance with the manufacturer's instructions, and assay was conducted based on, as a 1 unit, the protein treatment concentration of absorbance showing ED50 (50% effective dose) concentration.

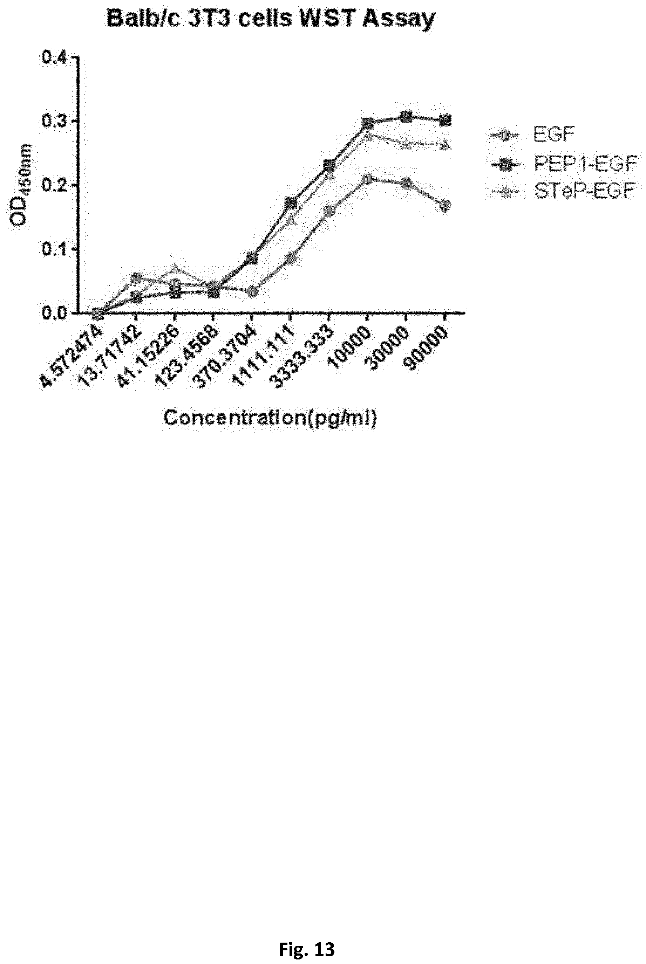

As a result, the purified EGF, PEP1-EGF and STeP-EGF exhibited cell proliferation activity of 6 X 10⁶ unit/mM or more. In particular, as compared to EGF, PEP1-EGF and STeP-EGF exhibited superior cell proliferation activity. This indicates that, although these recombinant proteins are fused with PTD, they have no problem of conjugation with EGF receptors in cells. FIG. 13 shows cell proliferation efficacy of EGF recombinant proteins (EGF, PEP1-EGF, STeP-EGF).

**TABLE 1**

| Cell proliferation activity (Unit) of EGF recombinant proteins | | | |
|---|---|---|---|
| Sample name | EGF | PEP1-EGF | STeP-EGF |
| Activity (unit/mM) | 6.27×10⁶ | 13.90×10⁶ | 14.34×10⁶ |

### [Example 11. Skin penetration analysis of cell- and skin-penetrating EGF proteins]

For skin penetration analysis of STeP-EGF by tissue staining, Franz cells using the porcine skin were treated with equal amounts (42 µM) of Normal EGF, PEP1-EGF and STeP-EGF. After 24 hours, the porcine skin between the Franz cell was taken out, washed with distilled water and subjected to tissue staining. The porcine skin site exposed to donor cells was cut and then immobilized in 10% buffered formalin to produce a paraffin block. Tissues were sectioned to 7 µm using a microtome. Then, the tissues were mounted on a slide, paraffin was removed, the residue was hydrated and tissue fragments were blocked for 30 minutes. The blocked tissues were treated with anti-EGF antibodies (Santa Cruz, green) and reacted at 4°C for 18 hours. On the following day, the resulting tissues were washed with PBST, treated with an Alexa Fluor 488 secondary antibody at room temperature for 2 hours and washed with PBST again. Then, for counter staining, the tissues were reacted with Hoechst 33342 (blue) at room temperature for 10 minutes, embedded in a fluorescence medium and observed with a fluorescence microscope.

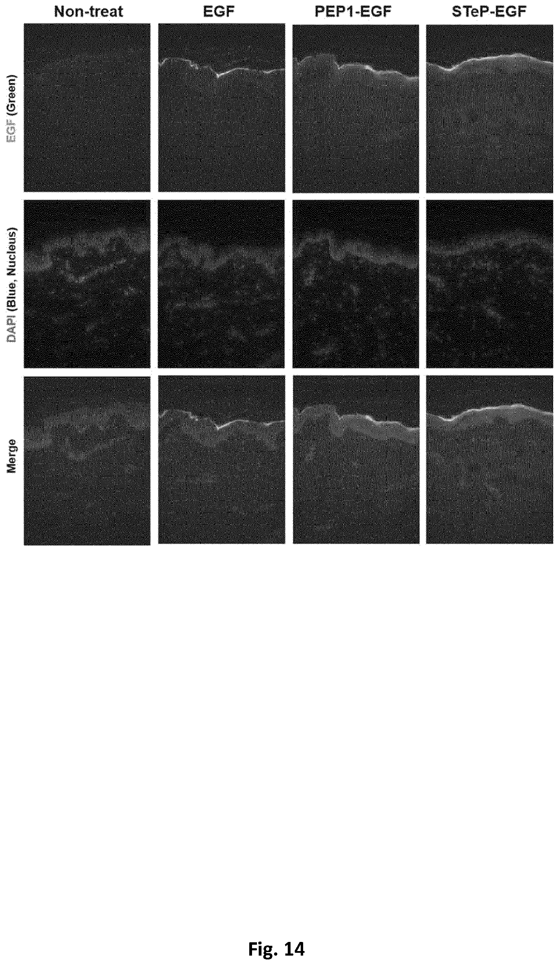

Test results showed that EGF was observed only in the cornified layer, while PEP1-EGF was observed even in the epithelial layer as well. It can be seen that the STeP-EGF of the present invention can be more efficiently delivered into the skin, as compared to PEP1-EGF. FIG. 14 shows comparison results of skin penetration capability of EGF proteins.

### [Example 12. Peptide synthesis]

In order to identify whether or not cell penetration capability can be improved through non-covalent bond between cell-penetrating peptides and target substances, the present inventors synthesized cell-penetrating peptides. The present inventors each synthesized PEP1 peptides, which were conventionally known to improve cell penetration capability through non-covalent bond, and novel cell-penetrating peptides, STeP. The peptide manufacturer was asked to synthesize the peptides in accordance with a liquid-solid method and sequences are shown in the following Table 2.

**TABLE 2**

| Peptide amino acid sequence | |
|---|---|
| **PTD** | **Amino acid sequence** |
| PEP1 | KETWWETWWTEWSQPKKKRKVC |
| STeP | KETWWETWWTEWSQPYGRKKRRQRRRC |

### [Example 13. Identification of cytotoxicity of cell-penetrating peptides]

Cytotoxicity was evaluated to identify whether or not the peptides of the present invention could be harmlessly used to deliver polymers *in vivo*.

HaCaT cells cultured in DMEM containing 10% FBS were seeded in a unit dose of 2.5×10⁴ on a 96-well plate, cultured for 24 hours and washed with HBSS twice. Cell-penetrating peptides, PEP1 and STeP, were dissolved at different concentrations of 50 µM, 100 µM and 200 µM in D.W. and diluted in a medium such that the final concentrations reached 2.5 µM, 5 µM and 10 µM, and the cells were treated with the resulting peptides and then reacted for 24 hours at 37°C in a 5% CO₂ incubator. After culture, the supernatant was removed, a WST solution was diluted in a medium in a ratio of 1:9 and the cells were treated with the medium. After culturing for 3 hours, absorbance was measured at 450 nm, and cell viability was calculated for cells of a peptide non-treatment group and cells of a peptide treatment group.

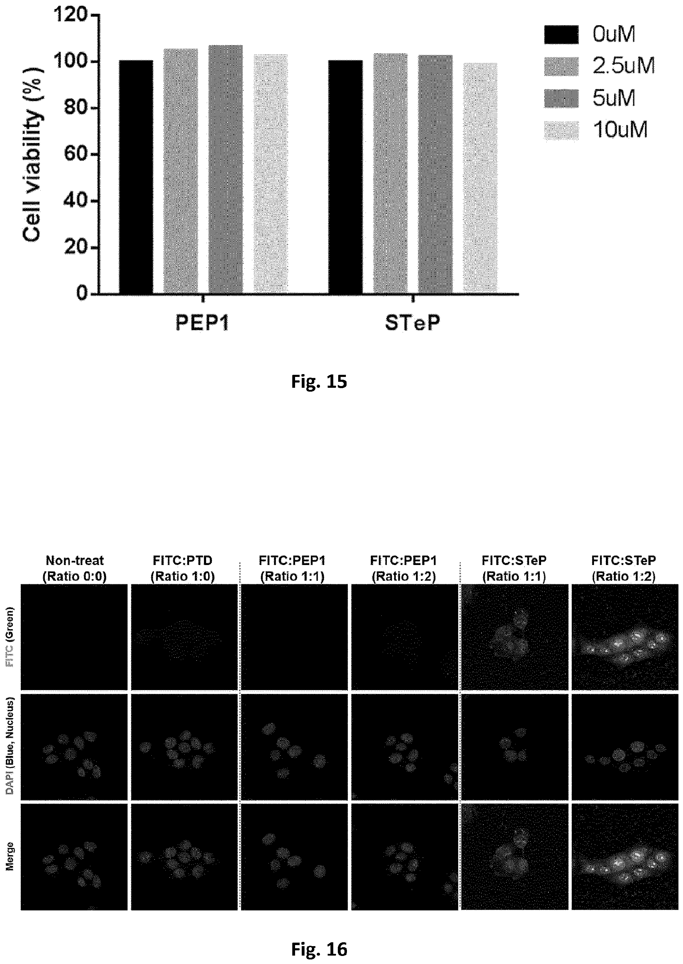

As a result, as can be seen from FIG. 15, both PEP1 and STeP did not exhibit cytotoxicity even at a concentration of 10 µM. FIG. 15 shows evaluation results of cytotoxicity of cell-penetrating peptides.

### [Example 14. Cell penetration analysis of cell-penetrating peptide-FITC mixture]

In the present Example, in order to easily analyze the capability of cell-penetrating peptides to deliver a target substance into cells, when the cell-penetrating peptides are non-covalently bonded to the target substance, a green fluorescence protein (fluorescein isothiocyanate: hereinafter, simply referred to as "FITC") was selected as the target substance.

FITC and cell-penetrating peptides (PEP1, STeP) dissolved in borax buffer (pH 8.0) were prepared. The FITC and cell-penetrating peptides (PEP1, STeP) were mixed in a molar ratio of 1:0, 1:1 and 1:2, and reacted at 37°C for 30 minutes, and cells were treated with 5 µM of the finally obtained cell-penetrating peptide-FITC mixture. After the cells were treated with the mixture, they were cultured for 90 minutes, the medium was added to the cells in the same amount as the mixture and the cells were further cultured for 30 minutes. After 30 minutes, the medium was removed, the cells were washed with PBS and were immobilized at -20°C with cooling methanol for 10 minutes. The cells were thoroughly washed with PBS, and dyed with a solution of a nuclear staining reagent (DAPI) diluted at 1/5000 in PBS in the absence of light for 10 minutes. The resulting cells were thoroughly washed with PBS, mounted and then analyzed with a confocal scanning laser microscope.

As a result, the group treated with the STeP-FITC mixture exhibited the brightest green fluorescence, and the green fluorescence was brighter in a molar ratio of 1:2, than in a molar ratio of 1:1. That is, as compared to PEP1, STeP further improved cell penetration capability of FITC. FIG. 16 shows comparison results of cell penetration capability of a cell-penetrating peptide-FITC mixture.

### [Example 15: Skin penetration analysis of cell-penetrating peptide-FITC mixture]

Franz cells (diameter of 9 mm) were used in order to identify whether or not penetration of FITC into the skin as well as cells was improved under the condition of non-covalent bonding to STeP.

FITC and cell-penetrating peptides (PEP1, STeP) dissolved in borax buffer (pH 8.0) were prepared. The FITC and cell-penetrating peptides (PEP1, STeP) were mixed in a molar ratio of 1:0 and 1:2, and reacted at 37°C for 30 minutes, and the top of Franz cells were treated with 100 µM of the finally obtained cell-penetrating peptide-FITC mixture. The porcine skin was isolated and washed with PBS three times. After washing, the cryo mold was treated with an OCT embedding matrix (Cell Path Ltd., UK) and tissues were frozen at -70°C. The frozen tissues were cross-sectioned to a thickness of 7 µm at -20°C using a cryotome (HM520, Germany) to produce fragment slides. To dye the nucleus of the skin cells, tissue fragments fixed to a slide glass were dyed with a solution of a nuclear staining reagent (DAPI) diluted at 1/5000 in PBS in the absence of light for 10 minutes and washed with PBS three times. The resulting cells were mounted and then analyzed with a confocal scanning laser microscope.

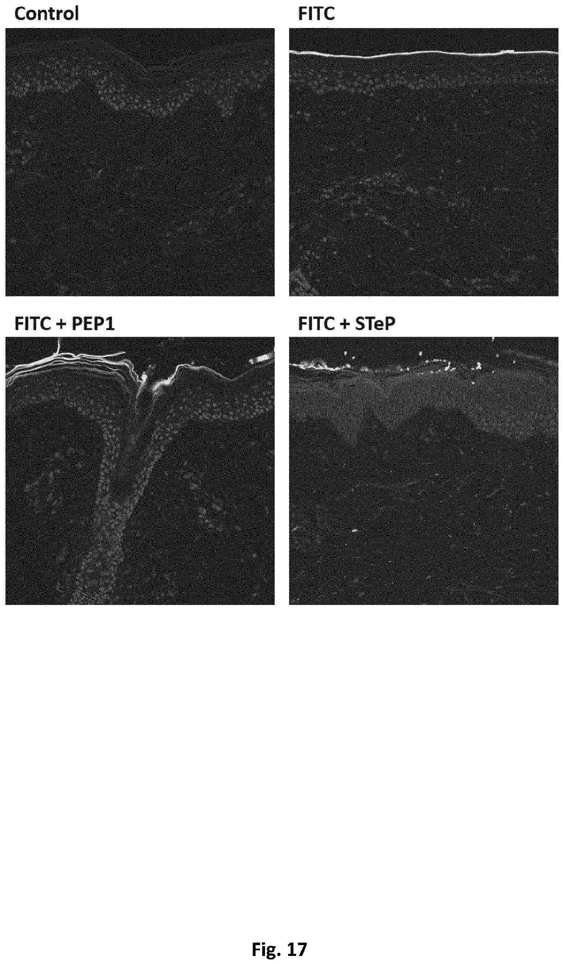

Test results showed that FITC was present in the cornified layer of the porcine skin, while the STeP-FITC mixture of the present invention could be efficiently delivered into the skin. FIG. 17 shows comparison results of skin penetration capability of the cell-penetrating peptide-FITC mixture.

### [Example 16. Cell penetration analysis of cell-penetrating peptide-(dipeptide diaminobutyroyl benzylamide diacetate) mixture]

In the present Example, use of the present invention as a cosmetic ingredient having anti-wrinkle efficacy was proved by identifying the improvement of physiological activity through skin penetration using peptides having skin physiological activity.

Dipeptide diaminobutyroyl benzylamide diacetate (DDBD, SYN-AKE®), which is known to contribute to the improvement of wrinkles by preventing signals to muscle contraction, was used as the peptide having skin physiological activity. DDBD was a peptide synthesized by mimicking the snake venom called "Waglerin 1".

In order to easily analyze the cell delivery capability of DDBD, FITC-fused DDBD (FITC-DDBD) was synthesized. To identify whether penetration of DDBD into cells was improved under the condition of non-covalent bonding to STeP, cell penetration of DDBD using FITC green fluorescence was observed in BALB/3T3 (mouse embryonic fibroblast cell line) cells.

BALB/3T3 cells were cultured on a slide for culture for 24 hours. Then, the culture medium (DMEM+10% BCS, 1% antibiotics) was removed, and the cells were washed with HBSS three times and treated with a mixture of STeP and FITC-DDBD. FITC-DDBD and STeP were mixed in a molar ratio of 1:5 and reacted at room temperature for 10 minutes, and the cells were treated with 10 uM of the finally obtained FITC-DDBD. After 60 minutes, the medium was removed, the cells were washed using HBSS and immobilized at -20°C using cooling methanol for 10 minutes. The cells were thoroughly washed with PBS and dyed with a solution of a nuclear staining reagent (DAPI) diluted at 1/5000 in PBS in the absence of light for 10 minutes. The resulting cells were thoroughly washed with PBS, mounted and then analyzed with a confocal scanning laser microscope.

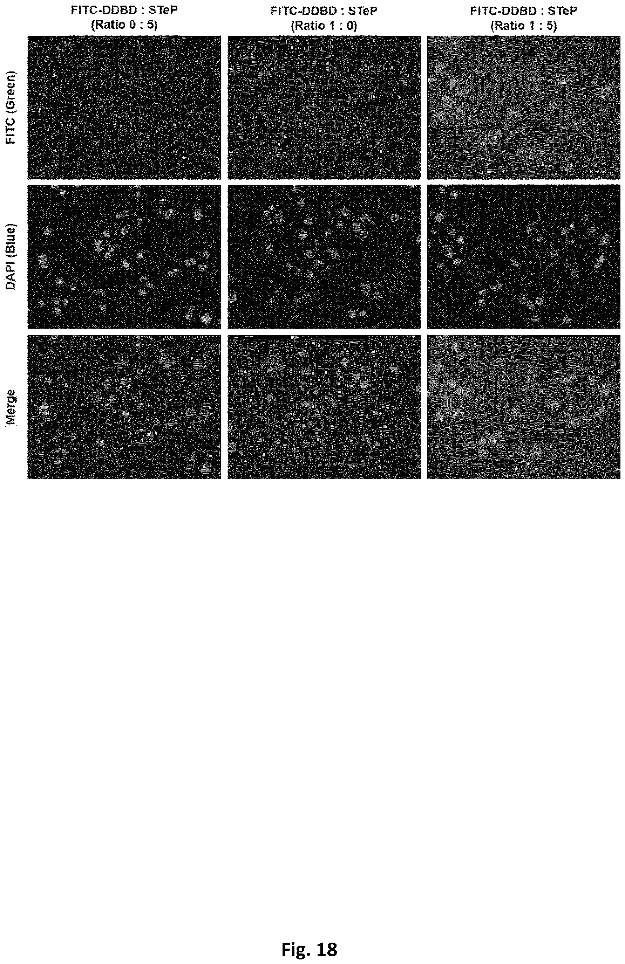

Results showed that, as compared to a group treated only with FITC-DDBD, a group treated with a combination of STeP and FITC-DDBD exhibited improved cell penetration. FIG. 18 shows comparison results of cell penetration capability of the cell-penetrating peptide/FITC-DDBD mixture.

### [Example 17. Skin penetration analysis of cell-penetrating peptide-FGF2 mixture]

In order to identify whether or not physiological activity through skin penetration was improved using large molecular-weight proteins as well as small molecular-weight peptide like DDBD, FGF2 (fibroblast growth factor-2, basic fibroblast growth factor) was used. FGF2 is known to regenerate the skin and recover healthy skin by stimulating keratinocytes and fibroblasts constituting the skin, but has difficulty penetrating the skin because it is a hydrophilic polymer substance.

For skin penetration analysis of FGF2 through tissue staining, Franz cells using the porcine skin were treated with equal amounts (500 µg) of FGF2, PEP1-FGF2 and a STeP-FGF2 mixture. After 24 hours, the porcine skin between the Franz cells was taken out, washed with distilled water and subjected to tissue staining. The porcine skin site exposed to donor cells was cut and then immobilized in 10% buffered formalin to produce a paraffin block, and tissues were sectioned to 7 µm using a microtome. Then, the tissues were mounted on a slide, paraffin was removed, the residue was hydrated and tissue fragments were blocked for 30 minutes. The blocked tissues were treated with anti-EGF antibodies (cell signaling, green) and reacted at 4°C for 18 hours. On the following day, the resulting tissues were washed with PBST, treated with an Alexa Fluor 488 secondary antibody at room temperature for 2 hours and washed with PBST again. Then, the tissue fragments were dyed with a solution of a nuclear staining reagent (DAPI) diluted at 1/5000 in PBS in the absence of light for 10 minutes and washed with PBS three times. The tissue fragments were mounted and then analyzed with a confocal scanning laser microscope.

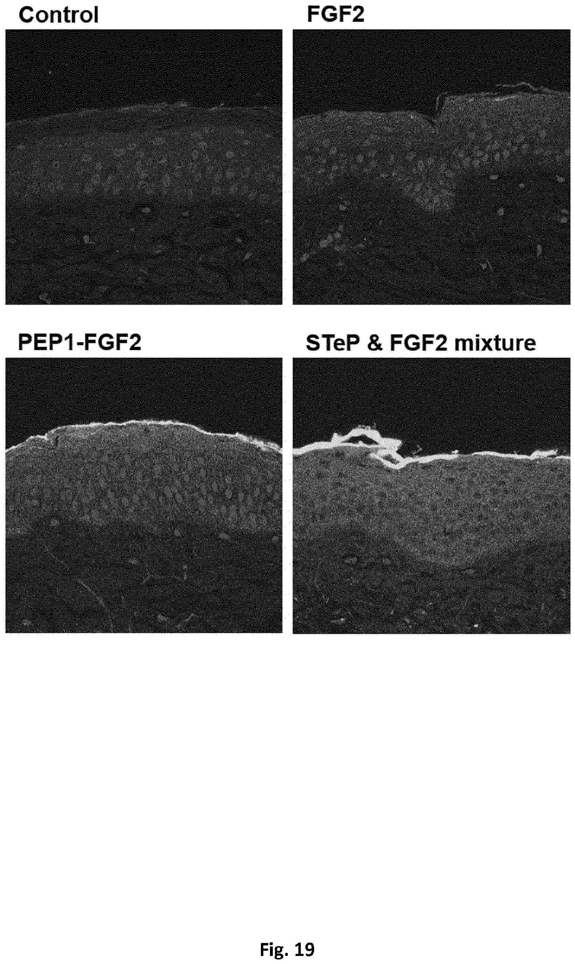

Results showed that FGF2 was not delivered into porcine skin tissues at all, while PEP1-FGF2 was efficiently delivered into the skin of the porcine skin tissues. In this case, although STeP-FGF2 was a simple mixture, it had better skin delivery capability than PEP1-FGF2. FIG. 19 shows comparison results of skin penetration capability of the cell-penetrating peptide-FGF2 mixture.

## Claims

1. A peptide having an amino acid sequence represented by SEQ ID NO: 1.

2. The peptide according to claim 1, wherein the amino acid sequence represented by SEQ ID NO: 1 is encoded by a nucleic acid sequence represented by SEQ ID NO. 2.

3. The peptide according to claim 1, wherein the peptide is a protein transduction domain (PTD).

4. The peptide according to claim 1, wherein the peptide is a gene delivery system.

5. A conjugate formed by bonding a substance that can be chemically bonded to a peptide having an amino acid sequence represented by SEQ ID NO: 1, to the amino acid sequence represented by SEQ ID NO: 1.

6. The conjugate according to claim 5, wherein the chemical bond is a covalent bond or a noncovalent bond.

7. The conjugate according to claim 6, wherein the substance that can be chemically bonded to the peptide is a peptide or protein.

8. The conjugate according to claim 6, wherein the noncovalent bond is an ionic bond or an electrostatic attraction-based bond or a hydrophobic interaction-based bond.

9. The conjugate according to claim 8, wherein the substance that can be bonded via an ionic bond or electrostatic attraction is a charged substance.

10. The conjugate according to claim 9, wherein the charged substance is DNA or RNA.

11. A complex formed by mixing a substance that can be bonded via hydrophobic interaction or electrostatic attraction to a peptide having an amino acid sequence represented by SEQ ID NO: 1, with the amino acid sequence represented by SEQ ID NO: 1.

12. The complex according to claim 11, wherein the substance that can be bonded via electrostatic attraction is a charged substance.

13. The complex according to claim 12, wherein the charged substance is DNA or RNA.

14. A cosmetic composition comprising:
a conjugate formed by bonding a substance that can be chemically bonded to a peptide having an amino acid sequence represented by SEQ ID NO: 1, to the amino acid sequence represented by SEQ ID NO: 1; or
a complex formed by mixing a substance that can be bonded via hydrophobic interaction or electrostatic attraction to a peptide having an amino acid sequence represented by SEQ ID NO: 1, with the amino acid sequence represented by SEQ ID NO: 1.

15. A pharmaceutical composition comprising:
a conjugate formed by bonding a substance that can be chemically bonded to a peptide having an amino acid sequence represented by SEQ ID NO: 1, to the amino acid sequence represented by SEQ ID NO: 1; or
a complex formed by mixing a substance that can be bonded via hydrophobic interaction or electrostatic attraction to a peptide having an amino acid sequence represented by SEQ ID NO: 1, with the amino acid sequence represented by SEQ ID NO: 1.

16. A method for introducing, into cells in a mammal, a substance that can be chemically bonded to a peptide having an amino acid sequence represented by SEQ ID NO: 1, comprising bonding the peptide having an amino acid sequence represented by SEQ ID NO: 1 to the substance that can be chemically bonded to the peptide having an amino acid sequence represented by SEQ ID NO: 1, to introduce, into the cells, the substance that can be chemically bonded to the peptide having an amino acid sequence represented by SEQ ID NO: 1.

17. A method for introducing, into cells in a mammal, a substance that can be bonded via hydrophobic interaction or electrostatic attraction to a peptide having an amino acid sequence represented by SEQ ID NO: 1, comprising bonding the peptide having an amino acid sequence represented by SEQ ID NO: 1 to the substance that can be bonded via hydrophobic interaction or electrostatic attraction to the peptide having an amino acid sequence represented by SEQ ID NO: 1, to introduce, into the cells, the substance that can be bonded via hydrophobic interaction or electrostatic attraction to the peptide having an amino acid sequence represented by SEQ ID NO: 1.


<110>    Bioceltran

<120>    Novel protein transduction domain and the usage thereof

<130>    YP-16-229

<150>    KR 10-2016-0148578
<151>    2016-11-09

<160>    2

<170>    KoPatentIn 3.0

<210>    1
<211>    27
<212>    PRT
<213>    Artificial Sequence

<220>
<223>    Protein transduction domain, STeP

<400>    1
Lys Glu Thr Trp Trp Glu Thr Trp Trp Thr Glu Trp Ser Gln Pro Tyr
  1               5                  10                  15

Gly Arg Lys Lys Arg Arg Gln Arg Arg Arg Cys
             20                  25

<210>    2
<211>    81
<212>    DNA
<213>    Artificial Sequence

<220>
<223>    Polynucleotide coding protein transduction domain, STeP

<400>    2
aaagaaacct ggtgggaaac ctggtggacc gaatggtccc agccgtatgg ccgtaaaaaa        60

cgtcgtcagc gtcgtcgttg t                                                  81